(19) Europäisches Patentamt
European Patent Office
Office européen des brevets

(11) EP 2 157 189 A1

(12) EUROPEAN PATENT APPLICATION

(43) Date of publication:
24.02.2010 Bulletin 2010/08

(51) Int Cl.:
C12Q 1/18 (2006.01)     B01L 3/00 (2006.01)
G01N 33/50 (2006.01)

(21) Application number: 08105007.2

(22) Date of filing: 11.08.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(71) Applicant: Koninklijke Philips Electronics N.V.
5621 BA Eindhoven (NL)

(72) Inventor: The designation of the inventor has not yet been filed

(74) Representative: Jungen, Carmen
Philips Intellectual Property &
Standards GmbH
Weißhausstraße 2
52066 Aachen (DE)

(54) **Apparatus and method for performing cellularsusceptibility testing**

(57) The present invention relates to a method and apparatus for performing cellularsusceptibility testing via a concentration gradient in a microfluidic device (200) including a microfluidic channel (202), comprising the steps of trapping one or more cells to be tested in one or more defined positions on an inner surface of the channel (202); applying a stable and predictable fluid concentration gradient through the channel (202) with the cells to be tested; incubating the cells within the concentration gradient, where, based upon the defined positions of the cells, an individual fluid concentration for each bacteria is known; and determining a concentration-dependent property of the cells.

FIG. 3

**Description**

FIELD OF THE INVENTION

The present invention relates to microfluidic systems. More particularly, the invention relates to a microfluidic apparatus and a corresponding method for a chemical gradient for performing cellularsusceptibility testing.

BACKGROUND OF THE INVENTION

The global increase in resistance of bacteria to antimicrobial drugs is generally recognized to be a growing threat for public health. Antibiotic susceptibility testing is commonly carried out in vitro to determine which antibiotic will be most successful in treating a bacterial infection and to avoid exposure to unnecessary antibiotics, which exacerbates the problem of antibiotic resistance. Currently, antibiotic susceptibility testing is carried out with methods based on bacterial culturing in liquid or solid media. Selective culture media can be used to grow only bacteria of a certain type in mixed populations. Several culture steps are often necessary to isolate a pure colony and produce a sufficient quantity of bacteria for subsequent analysis. Besides determining whether an organism is susceptible or resistant to a specific antibiotic, it is essential to investigate the dependence of the response on the antibiotic concentration. This is expressed by parameters such as the as minimum inhibitory concentration (MIC) and minimum bactericidal concentration (MIB). To determine which antibiotic is likely to eradicate an infection most quickly, it is also important to determine the time response of the bacteria to the antibiotic, i.e., to determine how fast the antibiotic can kill the bacteria or inhibit their growth.

Antibiotic susceptibility is usually measured with the Kirby-Bauer method. In this method, an agar plate is uniformly inoculated with the test organism and a paper disk impregnated with a fixed concentration of an antibiotic is placed on the agar surface. Growth of the organism and diffusion of the antibiotic take place simultaneously resulting in a circular zone of inhibition in which the amount of antibiotic exceeds inhibitory concentrations. The diameter of the inhibition zone is a function of the amount of drug in the disk and susceptibility of the microorganism. This test must be rigorously standardized since the zone size is also dependent on inoculum size, medium composition, temperature of incubation, excess moisture and thickness of the agar. If these conditions are uniform, reproducible tests can be obtained and the zone diameter is only a function of the susceptibility of the test organism. This allows the designation of an organism as "susceptible", "intermediate", or "resistant" to a certain concentration of antibiotic. The inhibition zone diameter can be correlated to the MIC by taking into account the diffusion rate of the antibiotic through the gel, or by calibrating the assay with susceptibility data measured by, e.g., agar and broth dilution methods.

For a more direct measurement of MIC, modified Kirby-Bauer assays have been developed which make use of strips with a gradient in the antibiotic concentration. Examples of these tests are the E-test (BD Biodisk) or the MICE test (Oxoid). Typically, 15-20 dilutions of an antibiotic are reformatted as a predefined concentration gradient on a plastic strip using dry chemistry technology. When applied to an agar plate where a microorganism is cultured, the strips create ellipses of microbial inhibition. The point at which the inhibition ellipse crosses the strip indicates the MIC.

While conventional methods for antibiotic susceptibility testing are powerful and versatile, they are time consuming processes. The methods require several rounds of culturing and incubation, leading to typical test times of 1-3 days depending on the microorganism. This is incompatible with the need for rapid diagnostics and often forces medical practitioners to use initial broad spectrum antibiotic treatment, which is costly, inefficient and increases the problems of antibiotic-resistant bacterial strains in hospital environments. For some infections, particularly those contracted by immuno-compromised patients in intensive care, the delay introduced by conventional methods can prove fatal to the patient.

In addition to the long time-to-results, methods based on gradient strips also have other disadvantages. For example, the bacterial growth line sometimes touches opposite sides of the same strip at different heights. This can be caused by handling errors, or imperfections in the agar causing uneven diffusion of the compound. If the MIC is higher than the maximum antibiotic concentration on the strip, no inhibition zone is visible. This can lead to the erroneous result that the tested bacterium is resistant to a certain antibiotic. Conversely, if the MIC is lower than the minimum concentration on the strip the growth does not intercept the strip, and the MIC cannot be determined.

To overcome the speed limitations of conventional microbiology methods, there is growing interest in methods that allow bacterial identification and antibiotic susceptibility testing on a single cell basis, which are made possible by recent progress in lab-on-chip technology and automated microscopy. The technology is based on immobilizing single bacteria on a surface in a microfluidic cartridge, exposing them to various antibiotics and monitoring their response over time. Fluorescent staining is used to differentiate between live and dead bacteria.

Single-cell approaches have several important advantages over conventional methods. The main advantage is that the test times needed for bacterial susceptibility testing are greatly reduced: antibiotic resistance can namely be determined in hours instead of days. This is due to the fact that the doubling time of single cells is in the order of 20-60 minutes, compared to the 12-48 hours generally required to visualize changes in macroscopic colonies. Since the individual cells are spaced on a surface, a high number of bacterial specimens from a mixed population can be analyzed simultaneously, contrary to traditional tests that require highly pure cultures. The method also allows for a detailed analysis of the temporal response of bacteria to antibiotics by measuring so-called "time vs. kill" curves. This information is not included in conventional MIC measurements.

Two methods are suggested for determining the MIC via a single cell approach:

1. Use different chambers for each concentration of antibiotic (parallel screening).
2. Increase the concentration in steps in the same chamber (serial screening).

Parallel screening has the disadvantage of requiring complicated microfluidics networks, as well as a large amount of bacteria in order to fill the different microfluidic chambers. Serial screening considerably increases the time needed for the assay, since the increase in antibiotic concentration must be slow compared to the doubling time of the tested microorganism, which is typically between 20-60 min. It is estimated that approximately 10-20 antibiotic concentrations are necessary for a sufficiently precise MIC determination.

Microfluidic devices have been recently recognized as powerful and versatile tools for generating chemical gradients. These devices greatly improve gradient stability and control compared to diffusion-based gradient generators, such as the Boyden, Zigmond and Dunn chambers. This has opened the door to a new type of bioassay in which the response of single cells is analyzed with unprecedented detail.

The simplest microfluidic device for generating a chemical gradient is a y-type channel structure (see, for example, F. Lin, E.C. Butcher, Lab Chip 6, 1462-1469 (2006)). In this device, a concentrated solution of test compound (e.g. antibiotic) is injected from one inlet and pure buffer is injected from the other inlet. A concentration gradient is formed in the channel when molecules of the test compound diffuse across the interface between the streams. In contrast to the conventional devices described above, the gradient is stable over time and can be maintained as long as fresh buffer and test compound solutions are supplied. The shape of the gradient is typically sigmoidal, but can be varied to some extent by adjusting the relative flows of solutions. The direction of the gradient can also be inverted by changing the inlet configuration.

The y-channel structure has however important disadvantages, which arise from the fact that the gradient formation relies on diffusion. The extension of the gradient is determined by the diffusion length $L_d = \sqrt{4Dt}$, where $D$ is the diffusivity (~1.7 x 10$^{-6}$ cm$^2$s$^{-1}$ for 10 kDa molecules) and $t$ is the residence time of the liquid in the system, i.e., $t = x / U$ where x is the position along the channel (as measured from the inlets) and U is the flow velocity. For characteristic values of x = 100$\mu$m and $U$ = 100$\mu$m/s, the diffusion length $L_d$ is in the order of 30$\mu$m. This means that a sharp gradient is present in the middle region of the channel or, in other words, that only a limited fraction of the channel width can be used in the assay. A smoother gradient can be obtained by increasing the permanence time (i.e., either by increasing the channel length or decreasing the flow velocity) so that $L_d$ becomes comparable to the width W of the channel. Due to the square root dependence, however, in order to increase the extension of the gradient by a factor of 10 the channel has to be 100 times longer (or the flow speed 100 times smaller). Long channels are not suitable for miniaturization, while low flow velocities introduce considerable time-delays when the gradient is applied, removed, or inverted.

Furthermore, the condition $L_d \approx W$ also implies that the gradient will evolve rapidly along the channel. Due to this, cells in different sections of the channel will experience different conditions. This can be avoided by limiting the test area to a single microscope field (in the order of 100 x 100 $\mu$m) over which the gradient does not change considerably, which however means that only a small fraction of the channel (and consequently only a small fraction of cells injected in the device) is analyzed.

To increase the efficiency of the assay, it would be beneficial to make use of a larger fraction of the channel. This involves sequential imaging of high power microscope fields, a task which can be easily accomplished with conventional microscopes equipped with an automated translation stage. A prerequisite for this is, however, a gradient which does not change significantly along the channel.

Furthermore, y-channel structures only allow creating gradients that are centered around 50% concentration. In other words, only the slope of the gradient can be varied but not its offset. In practice, however, one would like to be able to expand a gradient around arbitrary concentration between 0% and 100%. For example, let's assume that a first preliminary MIC measurement with a gradient varying from 0% to 100% indicates a MIC around 30%. To determine the MIC more precisely, one would like to expose the subsequent bacterial sample to a gradient centered around this value (for example 25% - 35%). This is not possible with conventional y-channel structures.

A three-channel microfluidic device for performing cellular susceptibility testing is disclosed in A Hydrogel-Based Microfluidic Device For The Studies Of Directed Cell Migration, Shing-Yi Cheng, et al., Lab On A Chip, 2007, 7, 763-769, which may include at least three parallel channels, including a center channel, a left channel and a right channel separated by a hydrogel medium, wherein the center channel provides a growth surface for bacteria, and wherein the hydrogel medium provides a means for nutrient supply to the bacteria via diffusion into the center channel. Fluids of different concentrations are introduced into the left channel and right channel, and the fluids diffuse through the hydrogel medium and into the center channel to form a concentration gradient in the center channel, so that a susceptibility of the bacteria to the introduced fluids may be observed.

To further address the drawbacks of the y-channel structure, microfluidic devices have been designed in which gradient generation relies on flow patterns rather than diffusion. Some of these devices are based on complicated networks of microfluidic channels, either in a branched configuration or by "tapping" from different positions in channels connecting two reservoirs. In these devices, the gradient shape can be controlled precisely and is defined by the inputs and the structure of the microfludic network. The gradient is essentially independent on the flow rate, and can be maintained over several millimeters along the detection chamber under laminar flow conditions.

The microfluidic network approach has however other drawbacks, depending on the structural arrangement utilized in the network. The network needed to create the gradient may occupy a considerable fraction of the device. The size of the network may be essentially proportional to the resolution of the gradient. As a result, the potential of the device for miniaturization and high throughput experimentation is limited. Moreover, the narrow microfluidic channels forming the network increase the chance of leakage and are easily blocked by entrapped air bubbles, debris, etc.

US 2003/0040119 Al discloses separation devices and methods for separating particles wherein a microfluidic device is used to sort particles, such as cells, in a solution. However, the disclosure does not teach a method to apply a stable and predictable fluid concentration gradient through the channel to determine a concentration-dependent property of the bacteria.

Thus, what is needed is a method and microfluidic device to create a stable concentration gradient of antibiotic, or another test compound, to measure cellular, e.g., bacterial, susceptibility. The method and device does not require complicated microfluidic networks and allows a direct and fast determination of MIC in a single microfluidic chamber. Because the variation in antibiotic concentration is spatial rather than temporal, the gradient can be very steep, thereby allowing a large range of concentrations to be screened. For a more sensitive determination of MIC, weaker gradients can be used.

## SUMMARY OF THE INVENTION

It is an object of the present invention to provide a method and apparatus that quickly and accurately measures cellular susceptibility to a variety of fluid concentration-dependent solutions, including antibiotics, pH conditions and ionic strength. The term "cellularsusceptibility" is defined as including toxicology, viability testing, and every other condition where cells, such as bacteria, may be affected by, or exhibit some effect from, a fluid concentration.

It is a further object of the present invention to provide a method and apparatus for quickly and accurately measuring cellularsusceptibility with a device that combines a high degree of gradient control and stability, and is simple and rugged.

According to a first aspect of the present invention there is provided a method for performing cellular susceptibility testing via a concentration gradient in a microfluidic device including a microfluidic channel. The susceptibility testing method includes the steps of trapping one or more cells to be tested in one or more defined positions on an inner surface of the channel; applying a stable and predictable fluid concentration gradient through the channel with the cells; incubating the cells within the concentration gradient, where, based upon the defined positions of the cells, an individual fluid concentration for each cell is known; and determining a concentration-dependent property of the cells. The cells may be bacteria trapped on the inner surface of the channel with an adhesive, e.g., a bacterial adhesive.

The apparatus and method for a creating a microfluidic antibiotic gradient for cellularsusceptibility testing allows precise (spatial and temporal) control over the concentration gradient. An important property is that the gradient is independent of the flow speed through the channel. Stable gradients can therefore be established in seconds using high flow speeds and do not decay over time, contrary to methods relying on diffusion. In one embodiment, the gradient is defined by the geometry of grooved structures in the device and can be maintained over several millimeters along the channel. Such a device is also much simpler than existing flow-based gradient generators based on complicated networks of microfluidic channels, and is less prone to blockage and leaking. An important application of the device is for a direct and fast determination of MIC or other concentration-dependent characteristics in a single microfluidic chamber. The apparatus can be manufactured relatively cheaply.

Advantageous embodiments of the method of the present invention, in particular of the ability to tailor the method to maximize the effectiveness of single-cell observations, are defined in the dependent claims.

In a preferred embodiment, at least one of the fluid solutions includes an antibiotic. The concentration gradient of the antibiotic or other solution may be dynamically adjusted by varying the concentrations of at least one of the two or more fluid solutions. These arrangements have the advantage of providing precise control over the specific concentration antibiotic required to affect one or more single cells under observation.

The cellular, e.g., bacterial, samples under observation may be incubated within the concentration gradient with a viability stain, thereby enabling the determination of the minimum inhibitory concentration and/or minimum bactericidal concentration from the defined positions of the bacteria within the stable and predictable antibiotic concentration gradient.

Many antibiotics do not directly kill microorganisms but simply prevent their growth. In this case, minimum inhibitory concentration (MIC) can be determined by measuring the ability of the bacteria to divide, or by using other markers of bacterial metabolic activity (e.g. $CO_2$ production).

The fluid solutions input through the device may be selected to provide a pH and/or ionic strength gradient, thereby providing information regarding additional concentration-dependent properties for the single bacterial cells within the concentration gradient.

The fluid solutions input through the device may be adjusted to flow into and through the channel at a steady and/or variable rate. Thus, the bacteria may be observed to determine a concentration-dependent adhesion and/or isoelectric property with respect to the channel.

The microfluidic channel may include two or more inlets for the introduction of fluids. Each of the two or more inlets may include at least one sub-inlet. These sub-inlets provide the advantage of permitting a wide range of solutions and concentrations to be introduced through the channel and to the bacterial samples.

The microfluidic device for creating the concentration gradient for cellularsusceptibility testing may include a means for trapping one or more single cells in one or more defined positions on an inner surface of the channel; a means for applying a stable and predictable fluid concentration gradient through the channel with the cells; a means for incubating the cells within the concentration gradient, wherein, based upon the defined positions of the cells, an individual fluid concentration for each cell is known; and a means for determining a concentration-dependent property of the cell.

The microfluidic device for performing bacterial susceptibility testing may further include at least three parallel channels, including a center channel, a left channel and a right channel separated by a hydrogel medium, wherein the center channel provides a growth surface for cells, and wherein the hydrogel medium provides a means for nutrient supply to the cells via diffusion into the center channel, wherein fluids of different concentrations are introduced into the left channel and right channel, wherein the fluids diffuse through the hydrogel medium and into the center channel to form a concentration gradient in the center channel, wherein a susceptibility of the cells to the introduced fluids is observed.

The microfluidic device for performing cellularsusceptibility testing may further include a third fluid supply channel mounted parallel to the right channel in the hydrogel medium; and a second cellulargrowth channel providing a second growth surface for cells mounted between and parallel to the right channel and the third fluid supply channel in the hydrogel medium, wherein upon the introduction of another fluid into the third fluid supply channel, a second concentration gradient is formed in the second cellulargrowth channel, wherein the susceptibility of the cells to the fluids forming the second concentration gradient is observed.

The microfluidic device for creating the concentration gradient may further comprise two or more channel segments between the inlets and the defined cell positions. In one embodiment, at least one segment includes a ridged element that includes a plurality of ridges in and/or on an interior channel wall, the ridged element being adapted to create a localized secondary flow effect and a local mixing effect in said at least one segment of the channel for the formation of said concentration gradient over the width of the channel. Each of the channel segments may include a dissimilar ridged element for creating localized secondary flow effects and local mixing effects in respective segments of the channel. Thus a stable and predictable concentration may be established quickly for introduction to the cellularsamples.

In additional embodiments, each segment may include a plurality of zones oriented parallel to fluid flow, e.g., nine zones, for the optimal arrangement of the ridged elements and the local flow and mixing effects. In at least a first segment type, there may be no ridged element in the three zones adjacent each side wall of the channel. In another arrangement, only a middle three zones include the ridged elements, and at least the first segment type includes the ridged element in only two of the three middle zones. Alternatively, the ridged element in the first segment may include a laterally staggered arrangement. Second and subsequent segments may include two rows of generally parallel ridged elements arranged side-by-side. The two generally parallel rows of ridged elements in each subsequent segment may be spaced farther apart than in a preceding segment. These arrangements provide the advantage of identifying the portions of the fluid stream which are subject to the secondary flow effects and local mixing effects, and include an orderly manner to methodically create a predictable gradient.

The height of the ridged element above the floor of the channel may be about 20-40% of the total height of the channel. The ridges of the ridged element may be angled with respect to the fluid flow direction. The angle may be between 30° to 60°. The first half of the ridged element in each segment may be angled clockwise from the fluid flow direction, and a second half of the ridged element in each segment may be angled counterclockwise from the fluid flow direction. In this manner, the exact physical embodiment of the ridged elements may be selected to match the properties of the fluids to create the desired gradient.

Various fluid chemicals may be introduced through the inlets, e.g., a concentrated antibiotic solution and a pure buffer solution. The dissimilar fluids will create a sharp gradient, while fluids having fewer concentration differences will form a more gentle gradient. Careful selection of the fluids provides the advantage of a gradient that is exactly appropriate for the testing to be accomplished.

A micro fluidic device according to the present invention includes numerous advantages. These advantages include the elimination of complicated fluidic structures. Further, the creation of the gradient in the microfluidic device is independent of the velocity of flow through the channel. The creation of a stable gradient allows for a direct and fast determination of MIC, or other concentration-dependent properties, in a single micro fluidic chamber. Also, the invention may integrate valves to reverse the gradient.

It shall be understood that the claimed device has similar and/or identical preferred embodiments as the claimed method and as defined in the dependent claims.

BRIEF DESCRIPTION OF THE DRAWINGS

These and other aspects of the invention will be apparent from and elucidated with reference to the embodiment (s) described hereinafter. In the following drawings

| | |
|---|---|
| Figs. 1A and 1B | show plan views of prior art microfluidic devices for producing a chemical gradient; |
| Figs. 2A and 2B | show plan views of an embodiment of a microfluidic device for producing a chemical gradient in accordance with an embodiment of the present invention; |
| Fig. 3 | shows a plan view of an embodiment of a microfluidic device for producing a chemical gradient in accordance with an embodiment of the present invention; |
| Fig. 4 | illustrates a plan view of MIC/MIB testing of a plurality of single bacterial cells in a concentration gradient in accordance with an embodiment of the present invention; |
| Figs. 5A and 5B | illustrate plan views of the testing of a concentration-dependent property of a plurality of single bacterial cells in a concentration gradient having a fluid flow in accordance with an embodiment of the present invention; |
| Figs. 6A and 6B | illustrate plan views of embodiments of a device for testing a concentration-dependent property of a plurality of single bacterial cells in a concentration gradient in accordance with an embodiment of the present invention; |
| Figs. 7-9 | illustrate plan views of embodiments of segments for a microfluidic device for producing a chemical gradient in accordance with an embodiment of the present invention; |
| Fig. 10 | illustrates a plan view of individual ridges for a microfluidic device for producing a chemical gradient in accordance with an embodiment of the present invention; |
| Fig. 11 | illustrates exemplary concentration profiles of two fluids at specific points along the channel of a microfluidic device in accordance with an embodiment of the present invention; and |
| Fig. 12 | illustrates exemplary concentration profiles of two fluids at specific points along the channel as a result of the localized mixing effects in accordance with an embodiment of the present invention. |

DETAILED DESCRIPTION OF THE INVENTION

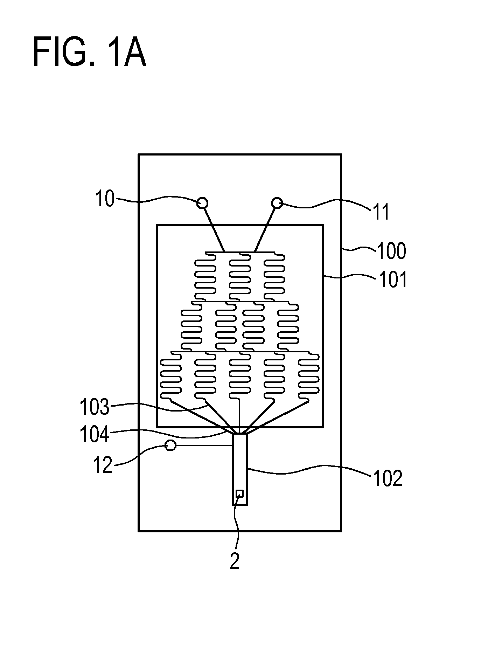

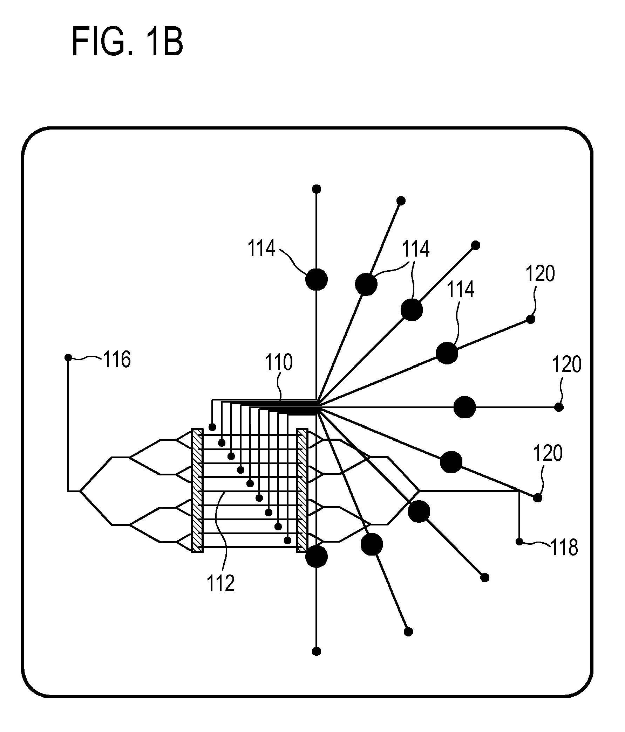

Figs. 1A and 1B illustrate prior art microfluidic devices which relies upon complex flow patterns to create a gradient generation. These devices are typically based on complicated networks of microfluidic channels, either in a branched configuration or by "tapping" from different positions in channels connecting two reservoirs. In these devices, the gradient shape can be controlled precisely and is defined by the inputs and the structure of the microfludic network.

Fig. 1A shows a schematic block diagram of a complex prior art device. In this example, the gradient chamber 100 includes a branch structure 101 and a monitoring chamber 102. The branch structure 101 has a plurality of interconnected branches (also called microfluidics) for mixing the solutions provided via the fluid inlets 10, 11, into said concentration gradient. Via a concentration gradient outlet 103 the generated concentration gradient is provided to a concentration gradient inlet 104 of the monitoring chamber 102, into which cells are loaded via the cell inlet 12. An electrical sensor 2 is arranged within said monitoring chamber 102.

Fig. 1B shows a schematic block diagram of another complex prior art device. In this example, the gradient chamber 110 includes a branch structure 112 and a number of monitoring chambers 114. The branch structure 112 has a plurality of interconnected branches for mixing the solutions provided via the fluid inlets 116, 118, into said concentration gradient. The generated concentration gradient is provided into the monitoring chambers 114, and then to the respective concentration gradient outlets 120.

These prior art microfluidic network approaches have significant drawbacks. The network needed to create the gradient occupies a considerable fraction of the device. The size of the network is essentially proportional to the resolution of the gradient. As a result, the potential of the device for miniaturization and high throughput experimentation is limited. Moreover, the narrow microfluidic channels forming the network increase the chance of leakage and are easily blocked by entrapped air bubbles, debris, etc.

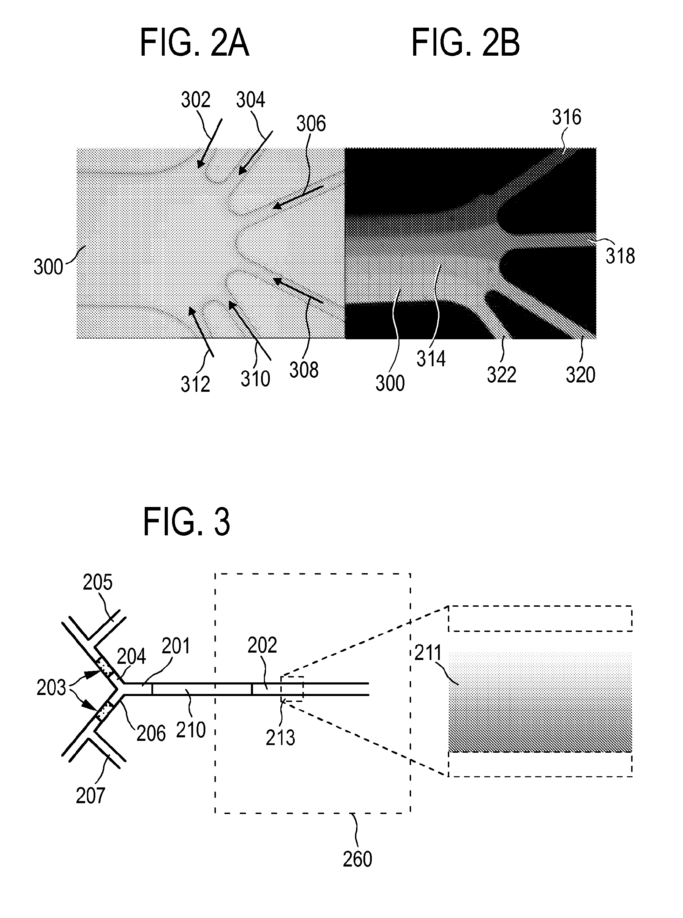

Figs. 2A and 2B illustrate planar views of modified Y-structures for producing a chemical gradient. Fig. 2A shows a gradient chamber 300 that includes six inlets 302, 304, 306, 308, 310, 312 for introducing six solutions of varying concentrations. For example, each of the solutions input may be identical with respect to the types of compounds comprising the solutions. However, the first inlet 302 may introduce the strongest solution concentration, the sixth inlet 312 may introduce the weakest solution, and the second through fifth inlets 304, 306, 308, 310 may introduce solution concentrations that are between the strongest and weakest solutions so as to create a concentration gradient 314 within the gradient chamber that is linear or nonlinear, stable and predictable, as desired for the concentration-dependent testing. Fig. 2B shows a modified Y-structure having a gradient chamber 300 that includes four inlets 316, 318, 320, 322. Notice that the concentration gradient 314 within the gradient chamber 300 is arranged in a linear manner based upon the logical introduction of incremental solution concentrations through the inlets 316, 318, 320, 322. Any combination of solution concentrations may be introduced into such systems as shown in Figs. 2A and 2B, limited only by the complexity of the modified Y-structures and the number of solutions desired for introduction through the inlets 302, 304, 306, 308, 310, 312, 316, 318, 320, 322. Logically, a more gradual and controllable concentration gradient may be created with a Y-structure having a large number of inlets and a large number of discrete solution concentrations. However, such an arrangement is quite complex.

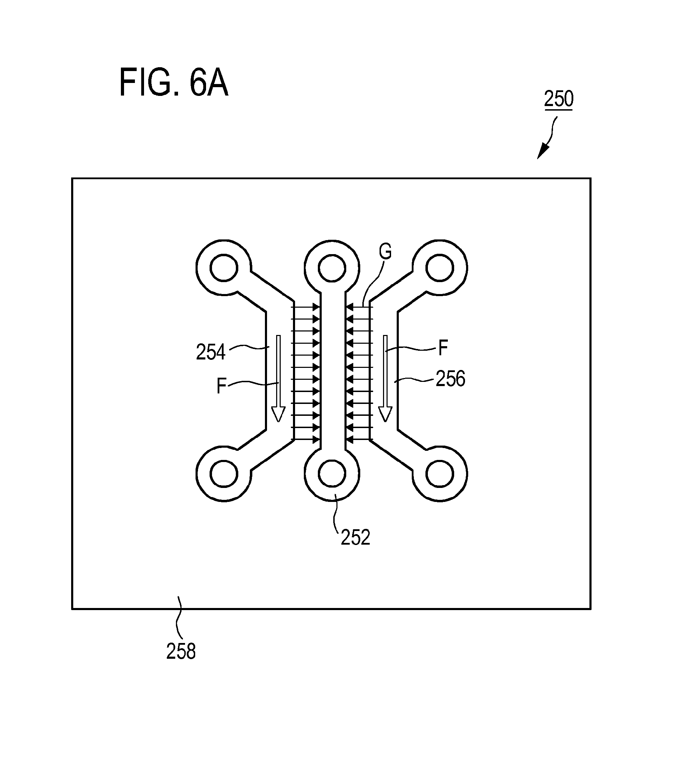

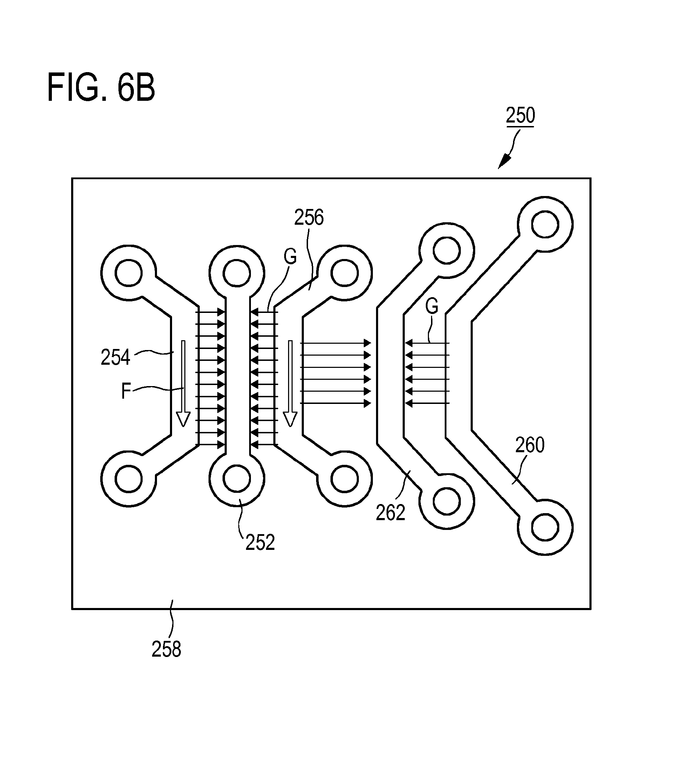

Fig. 3 shows a plan view of an embodiment of a microfluidic device for producing a chemical gradient in accordance with an embodiment of the present invention. At the heart of the device shown is a simple Y-structure 201. It is simple because it has only two inlets 204, 206. Each of the inlets 204, 206 may include a sub-inlet 205, 207 for introducing a second fluid through the inlets 204, 206 and into the channel 202. Of course, the sub-inlets 205, 207 in effect create an additional level of Y-structures where fluids may be mixed and/or gradients may be created. A mixer segment 203 may be included within the inlets 204, 206 for mixing the input solutions to the desired degree. In addition, the channel 202 may include one or more ridged elements 210 for creating a stable and predictable concentration gradient 211 across the width W of the channel 202 in an observation area 213. The ridged elements 210 are described in greater detail with respect to Figs. 6-9 below.

As an example of the operation of such a microfluidic device, a concentrated solution of, e.g., antibiotic is injected from one inlet 204 and pure buffer is injected from the other inlet 206. A gradient 211 is formed in the channel 202 when the antibiotic diffuses across the interface between the antibiotic and buffer streams. The concentration gradient is stable over time and can be maintained as long as fresh buffer and antibiotic solutions are supplied. The shape of the gradient is typically sigmoidal, but can be varied to some extent by adjusting the relative flows of the inlets 204, 206. The steepness of the gradient 211 can be varied by increasing/decreasing the flow symmetrically at both inlets. By using asymmetric flows, the location of the midpoint (50% concentration) in the channel can be varied. The gradient can also reversed by swapping the connections at the inlets.

In a conventional y-channel structure, only gradients that are centered around 50% concentration can be created. In other words, only the slope of the gradient can be varied but not its offset. In practice, however, one would like to be able to expand a gradient around an arbitrary concentration between 0% and 100%. For example, a first preliminary MIC measurement with a gradient varying from 0% to 100% indicates a MIC around 30%. To determine the MIC more precisely, one would like to expose the subsequent bacterial sample to a gradient centered around this value, e.g., 25% - 35%. This is not possible with a simple Y-structure.

To overcome this limitation, the Y-structure may be equipped with two additional inlets 205, 207. In this scheme, a concentrated solution of antibiotic (100%) is injected from inlets 204, 205, while pure buffer is injected from the other two inlets 206, 207.

The flows F at the various inlets are chosen so that the total flow is constant:

$$F_{A1} + F_{A2} = F_{B1} + F_{B2} = 1 \; (\text{normalized})$$

The choice $F_{A1} = 1$, $F_{A2} = 0$, $F_{B1} = 1$, $F_{B2} = 0$ corresponds to the simple case of a Y-channel structure. The gradient direction can be reversed by using the settings $F_{A1} = 0$, $F_{A2} = 1$, $F_{B1} = 0$, $F_{B2} = 1$.

By choosing the flows as follows:

$$F_{A1} = x, \; F_{A2} = 1\text{-}x$$

$$F_{B1} = 1\text{-}y, \; F_{B2} = y$$

with x and y between 0 and 1, any arbitrary concentration range between 0% and 100% can be addressed. The maximum and minimum antibiotic concentration in the test chamber are then

$$c_{max} = (100^*x)\%$$

$$c_{min} = (100^*y)\%$$

In one embodiment, in order for the device to function properly, the concentrations of the fluids in the two inlets 204, 206 at the entrance of the channel 202 should be homogeneous. Therefore, the device could be equipped with microfludic mixer structures 203 in each inlet 204, 206 (Fig. 3) to make sure that the flow streams entering from the inlets 204, 205, 206, 207 are well mixed.

The flows at the various inlets 204, 205, 206, 207 can be varied directly with external means, e.g., with different syringe pumps. A more integrated approach may consist in applying a constant pressure and varying the resistance in the channel 202, e.g., tunable microtopographies (microfluidic resistors) as disclosed by Lam, E.W., Cooksey, G.A., Finlayson, B.A., and Folch, A., "Microfluidic Circuits with Tunable Flow Resistances", Applied Physics Letters (2006), 89, 164105 (2006).

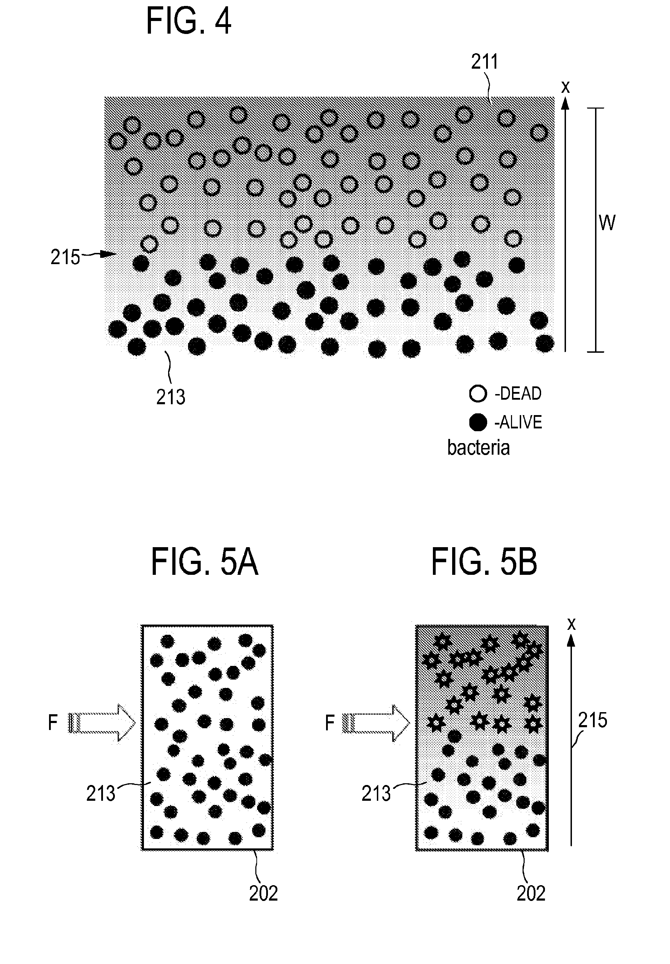

Fig. 4 illustrates a plan view of MIC/MIB testing of a plurality of single bacterial cells in a concentration gradient 211 in the observation area 213. The stable and predictable concentration gradient was created and introduced through the channel 202 and into the observation area/bacteria trap 213 where a number of individual bacteria •,○ are placed. The concentration gradient 211 in this example is established with an antibiotic solution to determine the MIC/MIB for the specific type of bacteria •,○. The highest concentration of antibiotic is at the top of Fig. 4 and the lowest antibiotic concentration is at the bottom. All of the bacteria •,○ started the testing process as live bacteria •. Upon exposure to the graduated antibiotic solution, some of the bacteria died to become dead bacteria o. Generally, the dead bacteria ○ are exposed to the high antibiotic concentration. The live bacteria • are generally in the lowest antibiotic concentration. The bacteria •,○ may be incubated with a viability stain which clearly shows which bacteria are dead ○ and which are alive •. The MIC/MIB 215 may be determined by locating the position along the width W of the channel demarcating the division between the live bacteria • and dead bacteria ○. The specific value for MIC/MIB 215 may be determined by interpolating between the starting concentrations of the antibiotic solutions introduced into the channel 202, based upon the relative position of the MIC/MIB 215 along the width W of the channel 202. The MIC/MIB 215 may be more precisely determined through another test procedure where the starting concentrations are closer to the MIC/MIB 215 derived from the previous test, so as to create a more gradual concentration gradient that is within a smaller range, i.e., a weaker gradient. Alternatively, concentrations of the solutions introduced through inlets 204, 206 and into the channel 202 may be adjusted to move the antibiotic concentration corresponding to the MIC/MIB through the remaining live bacteria •, to perform a quick verification. Of course, some of the live bacteria • will become dead bacteria o upon exposure to an antibiotic concentration above the MIB/MIC concentration.

As discussed above, various microfluidic approaches have been demonstrated for the generation of concentration gradients. Gradients can be obtained by means of diffusion, flow patterns, or a combination or both. For this application, a particularly suitable device employs a number of ridged elements 210 to create stable and predictable concentration gradients suitable for bacterial testing. The device has several advantages: it allows a precise (spatial and temporal) control over the chemical gradient. The gradient is independent of the flow speed, is entirely defined by the geometry of the structure, and can be maintained over several millimeters along the channel under laminar flow conditions.

Due to these properties, the concentration profile across the channel can be easily predicted and does not require knowledge of the diffusion rate of the antibiotic. The MIC/MIB 215 can therefore be determined by the coordinate *x* of the boundary between live and dead bacteria or, respectively, between bacteria that are dividing and bacteria showing inhibition of growth.

In addition to antibiotics, the approach described above can be extended to concentration gradients of any compound affecting the viability of cells either directly or indirectly (i.e. via a less favorable environment). This additional information could be used in many ways, e.g. to identify the tested microorganism more precisely.

In some cases, it can be beneficial to adjust the gradient dynamically. This includes varying the slope of the gradient or its offset. This can be easily achieved by varying the concentration of antibiotic or other solution or compound at one or both of the inlets 204, 206.

Dynamic adjustment of the gradient has at least a twofold purpose:

i. To ensure that the MIC is within the concentration range covered.
ii. To increase the resolution of the MIC determination by expanding a concentration range of interest.

As explained above, the fact that variation in antibiotic concentration is spatial (rather than temporal) permits the use of very steep gradients. This increases the dynamic range of the device, and allows a wide range of concentrations to be tested in a single incubation step. To determine MIC more precisely, a weak gradient can be established in the concentration range of interest.

Alternatively, a very sharp concentration gradient could be used to expose half of the bacteria in the test volume to a fixed concentration of antibiotics, and use the rest as a negative control.

Figs. 5A and 5B illustrate a plan view of the testing of a concentration-dependent property of a plurality of single bacterial cells in a concentration gradient having a fluid flow. In this embodiment, bacterial adhesion is measured with fluid flow F. The adhesion is modulated by means of an applied gradient in pH or ionic strength. In addition to viability, chemical gradients can also be used to induce changes in other cell functions. For example, bacterial adhesion could be modulated by establishing a gradient in pH or ionic strength in the test chamber. Both properties have been shown to have a strong influence on bacterial adhesion. The information gained from these tests could be added to the panel of parameters used in the identification of the tested microorganism.

Biological solutions in general contain many different ions, e.g., Na+, K+, Ca2+, Cl-, HCO3-, etc... The "ionic strength" is the total concentration of all these ions, corrected for their valence. The surface charge of bacteria, and hence their adhesion properties, has also been shown to change upon absorption of specific cations, e.g., Ca2+, Sr2+, Ba2+, even when the total ionic strength is kept constant. With respect to a pH gradient, the pH can be varied by adding a small quantity of essentially any acid or base. For example, HCl may be used as an acid and KOH and NaOH as bases.

In case of flow-based gradient generators, such as the one described below, the degree of bacterial adhesion can be probed by means of the fluid flow naturally present in the test volume. For this purpose, the number of cells in the test volume is monitored over time under a constant, or variable, if necessary, fluid flow, see Figs. 5A and 5B. When the adhesion of a microorganism is reduced below a certain threshold, the microorganism is removed from the surface by the drag force of the fluid flow and carried away with the flow. By measuring the extension of the cell-free region and taking into account the flow profile across the channel, the degree of adhesion of the tested microorganism could be determined quantitatively.

A gradient in pH could also be used in combination with electrical trapping of bacteria to measure their isoelectric point. Bacterial cells possess in general a net charge and can therefore be trapped by applying a DC electric field. A pH gradient can be used to vary the net charge of the bacteria across the test volume. At the isoelectric point, the bacteria will have no net charge. They will then be unaffected by the field and will be removed by the fluid flow.

Fig. 5A illustrates a population of cells • adhering in the channel 202 against the fluid flow F in the absence of a pH or ionic strength gradient. Fig. 5B illustrates the effects of a pH or ionic strength gradient on the adhesion properties of the cells •. A stable and predictable concentration gradient was created and introduced through the channel 202 and into the observation area 213 where a number of individual bacteria • are placed. The concentration gradient 215 in this example is established with respect to the pH or ionic strength of the solution to determine the cellular adhesions parameters the specific type of cell •. The highest ionic concentration/pH of solution is at the top of Figs. 5A and 5B and the lowest concentration/pH is at the bottom. All of the bacteria • started the testing process adhering in the channel 202. However, upon exposure to the graduated ionic strength/pH solution, some of the cells lose their adhesion properties and are removed by the fluid flow F. The removed cells are represented as ☼ .

In this example, the removed cells ☼ are exposed to the high pH/ionic strength solution, and remaining cells • are in the lowest pH/ionic strength solution. However, the adhesion characteristics for the cells • would vary depending on the type of cell. The specific value for pH/ionic strength characteristics of the cells • may be determined by locating the position along the width W of the channel demarcating the division between the remaining cells • and removed cells

☼ The pH/ionic strength values may be more precisely determined through a subsequent test procedure where the starting concentrations are closer to the derived pH/ionic strength values derived from the previous test, so as to create a more gradual concentration gradient that is within a smaller range, i.e., a weaker gradient. Alternatively, the pH/ionic concentrations of the solutions introduced through inlets 204, 206 and into the channel 202 may be adjusted to move the pH/ionic concentration corresponding to the derived value into the field of adhering bacteria • to perform a quick verification of the original value. Of course, some of the adhering bacteria • may become negatively affected by adjusted pH/ionic solution and may become swept away in the fluid flow F.

Due to these properties, the concentration profile across the channel 202 can be easily predicted and does not require knowledge of the diffusion rate of the solution, whether antibiotic, pH, ionic strength or other parameter. The threshold concentration for MIC/MIB 215, pH, ionic strength, or other concentration-dependent characteristic can therefore be determined by the coordinate *x* of the boundary between live and dead/removed bacteria or, alternatively, between bacteria that are dividing and bacteria showing inhibition of growth.

Fig. 6A illustrates a plan view of the testing of a concentration-dependent property of a plurality of single bacterial cells in a three-channel device mounted in a hydrogel medium. A number of experiments were performed to confirm that bacteria can grow and divide inside microfluidic device, where they could also be treated with different antibiotics. In one experiment, a three-channel gradient device 250 (see Fig. 6A), of a type which is also used for chemotaxis experiments with human cells was employed. Such a device 250 includes a means for trapping (213) one or more single bacteria in one or more defined positions on an inner surface of the channel (202); a means for applying a stable and predictable fluid concentration gradient (210, 216, 218, 220) through the channel (202) with the bacteria; a means for incubating (260) the bacteria within the concentration gradient, wherein, based upon the defined positions of the bacteria, an individual fluid concentration for each bacteria is known; and a means for determining a concentration-dependent property (215) of the bacteria. The device described may include three channels, including one center channel 252 and two outer channels 254, 256. The three channels 252, 254, 256 were mounted in and separated by a hydrogel medium 258, which provided a surface for the bacteria to grow on, and where nutrients could be supplied via diffusion through the side wall gels, without actually having a liquid flow on top of the bacteria. In addition, such a gradient device can be used to create a gradient in antibiotic concentration, which would make it possible to immediately not only test antibiotic susceptibility as a YES or NO question, but also to determine the actual minimum inhibitory concentration (MIC) necessary to stop growth or kill the bacteria.

In one experiment, Escherichia coli (E coli) K12 was used as the model bacteria. 50 $\mu$l of an overnight culture of E coli was re-suspended in 5 ml Brain-Heart-Infusion (BHI) growth medium. The new suspension was incubated in 37 ˚C for 1.5 - 2 hours, and thereafter the E coli were considered to be in or reasonably close to an exponential growth phase. Growth in a flow was tested in an ordinary micro fluidic flow chamber on a glass substrate with a silane amino-coating. The bacteria were allowed to stick on the surface and thereafter they where continuously flushed with BHI at 2 $\mu$l/min. In all the experiments, growth was studied in an ordinary microscope, on top of a 37 ˚C stage heater. The bacteria that were stuck on the surface can indeed grow, but some were swept away with the flow and many 'daughter cells', as a product of cell division, do not adhere to the surface and therefore also disappear in the flow. This shows that the conditions in the microfluidic device 200, 250 on the microscope heating stage are sufficient to get a rapid growth of the bacteria, but even if the cells are initially stuck to the surface, it is difficult to keep them and their 'daughter cells' on the surface for a longer time.

As a next step, E coli were instead allowed to grow in the middle channel 252 of a hydrogel gradient device 250. The outer channels 254, 256 were flushed F with BHI growth medium G, which was supplied through the hydrogel medium to the middle channel 252. Since there was no flow in the middle channel 252, where the bacteria grew, the bacteria did not have to attach to the surface but could instead grow in suspension inside the microfluidic chamber 252.

The results revealed that the gradient device 250 is well suited to create a good flow-free environment with a steady supply of nutrients G. This arrangement allowed the E coli to divide very quickly and such a setup is therefore well suited for antibiotic testing, either in a concentration gradient for MIC determination or with a uniform concentration for a simpler YES or NO assay. To test the idea of growing bacteria in an antibiotic gradient, the surface of the gradient device was covered with polylysine, a well-known bacterial adhesive layer. Polylysine promotes adhesion of the bacteria to the surface, which make the characterization easier and the measurement less sensitive to small flows that may occur in the middle channel 252.

In order to create an antibiotic gradient in the middle channel 252, one of the outer channels 256 was flushed with BHI + 0.2 $\mu$g / ml cefotaxime. The left outer channel 254 contained only BHI. Cefotaxime is a very efficient antibiotic, which inhibits regeneration of the cell wall and therefore over time creates bacterial lysis. The MIC for cefotaxime of the E coli K12 should be around 0.125 $\mu$g / ml. The preliminary estimations, deduced from the characterization of the chemotaxis device and the starting concentrations of the solutions, indicated that the MIC concentration would indeed be within the middle channel 252 under these conditions.

From the observations a few hours later, an antibiotic gradient was apparent within the middle channel 252, and it was possible to estimate a part of the middle channel 252 that was below the MIC. Initially, the bacteria grow as usual, but after some time, the E coli that are exposed to higher concentrations of cefotaxime start to die (lyse).

The experiments support the principles for using a concentration gradient device 200, 250 to treat growing bacteria with a gradient in antibiotic concentration. Such a device would make it possible to quantitatively determine the antibiotic susceptibility within a few hours instead of days. Naturally, this would require a proper calibration of the concentration gradient and a large enough concentration span. To get a logarithmic concentration span with the gel device 250, one could assemble several parallel channels with gels in between. The highest concentration on one side of a first measurement channel could be the lowest concentration on the other side of second measurement channel, etc. Also, an adhesive coating may make the flow-based devices 200 easier to use.

In another embodiment, see Fig. 6B, the gradient device 250 may be expanded, wherein, in addition to the three parallel channels, including a center channel (252) a left channel (254) and a right channel (256) mounted in a hydrogel medium (258), a third fluid supply channel 260, similar to left and right channels (254, 256) is mounted parallel to the right channel (256) in the hydrogel medium (258). A second bacterial growth channel 262, similar to the center channel (252), is mounted between and parallel to the right channel (256) and the third fluid supply channel 260 in the hydrogel medium (258) which provides a second growth surface for bacteria. Upon the introduction of another fluid into the third fluid supply channel 260, a second concentration gradient is formed in the second bacterial growth channel 262, wherein the susceptibility of the bacteria to the fluids forming the second concentration gradient is observed. The second concentration gradient is formed from the fluids introduced into the right channel (256) and the third fluid supply channel 260.

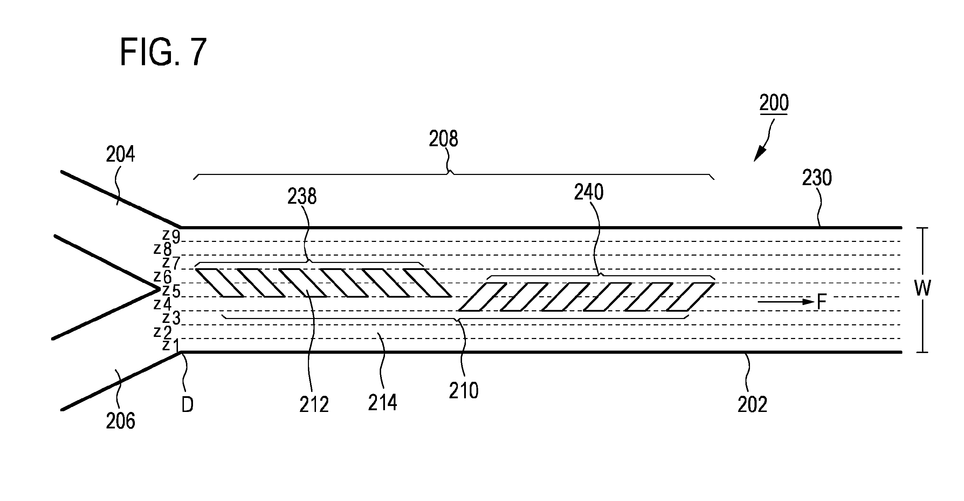

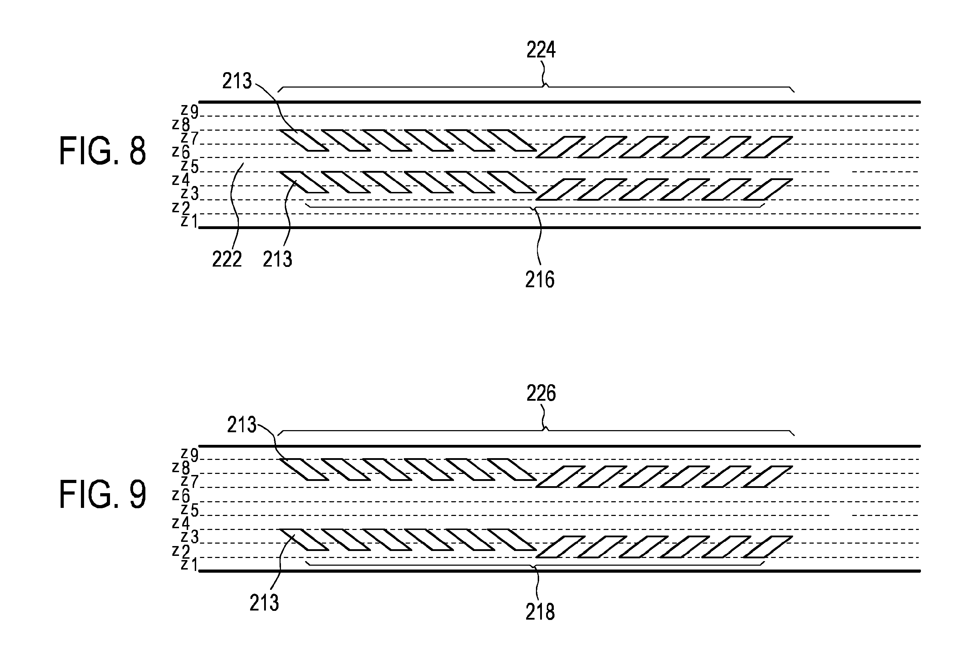

Fig. 7 shows a planar view of an embodiment of a microfluidic device 200 for producing a chemical gradient in accordance with embodiments of the present invention. The microfluidic device 200 includes a microfluidic channel 202 having two or more inlets 204, 206 for introducing fluid solutions, two or more channel segments 208, wherein at least one segment 208 includes a ridged element 210 including a plurality of ridges 212 in an interior channel wall 214 The ridged element 210 is adapted to create a localized secondary flow effect and a local mixing effect in that segment 208 of the channel 202 for the formation of said concentration gradient over the width W of the channel. Only a single segment 208 is shown for clarity. However, the channel 202 may include a number of segments 208, 224, 226, 228. Each of the two or more channel segments 208, 224, 226, 228 may include a dissimilar ridged element 214, 216, 218, shown in Figs. 8-10, for creating localized secondary flow effects and local mixing effects in respective segments of the channel 202.

The ridged element 210, 216, 218, 220 may be included in a channel floor 214. Some of the ridged elements 216, 218, 220 may include a formation of at least two parallel ridges 212 separated by a groove 222 that is substantially even with a channel floor 214. With respect to a width W of the channel 202 perpendicular to fluid flow direction F, the ridged element 210 may be in a middle portion of a channel floor 214 only. This arrangement is illustrated in Fig. 7.

With respect to a fluid flow direction F perpendicular to the width W of the channel 202, the segments 208, 224, 226, 228 may include a plurality of parallel zones Z1-Z9 for the purpose of illustrating the various fluid concentrations within the channel 202. Figs. 7-10 illustrate nine parallel zones each, but the number of zones is arbitrary and is intended to aid in understanding the function of the device 200.

As illustrated in Fig. 7, the segment 208 does not include a ridged element 210 in the three zones Z1-Z3, Z7-Z9 adjacent each side wall 230 of the channel 202. This segment 208 illustrates that the ridged element 210 may be in only the middle three zones Z4-Z6. In addition, the ridged element 210 may include two distinct arrangements 232, 234 that are staggered or offset with respect to the width W of the channel 202. The segment 208 may include the distinct portions 232, 234 of the ridged element 210 in only two of the three middle zones Z4-Z6 each. Thus the arrangement of Fig. 8 may provide fluid mixing with respect to a central portion of the fluid flow through the channel 202.

The grooved/ridged element 210 illustrated in Fig. 7 is arranged such that mixing occurs, with respect to the width W of the channel, only in the zones corresponding to the location of the ridged element 210. The ridged elements 210, 216, 218, 220 are oriented at a certain angle $\alpha$ with respect to the main flow direction F. The angle $\alpha$ illustrated is about 45˚, but it could be between 30˚and 60˚. The width direction of the first arrangement of ridges 238 may be about 2/3 the total width of zones for the entire ridged element 210. In one embodiment, the width of the first arrangement of ridges 238 is about 111 $\mu$m. The height of the ridges 236 or depth of the grooves may be between 20 and 40% of the total channel height, which may be about 25 $\mu$m. The dimensions of each ridge 236 in a streamwise direction may be comparable to the channel height, which may be about 50 $\mu$m. The pattern shown in Fig. 7 consists of two distinct arrangements 238, 240 of repeating grooves/ridges, oriented at 45˚ and -45˚ with respect to the main flow F, and displaced or shifted somewhat perpendicular to flow direction F.

The pattern of grooves/ridges comprising a ridged element 210, as depicted in the Fig. 7 may be called one "cycle" and may consist of a first arrangement of ridges 238, e.g., six "type 1" grooves/ridges, followed by a second arrangement of ridges 240, e.g., six "type 2" grooves/ridges. In practice, the number of repeated grooves/ridges of a certain type may be smaller or larger, and may be typically between 5 and 10. The "cycle" of Fig. 7 may be repeated several times, i.e., between 5 and 20 times, to obtain a good homogenization of the fluids within the desired zones.

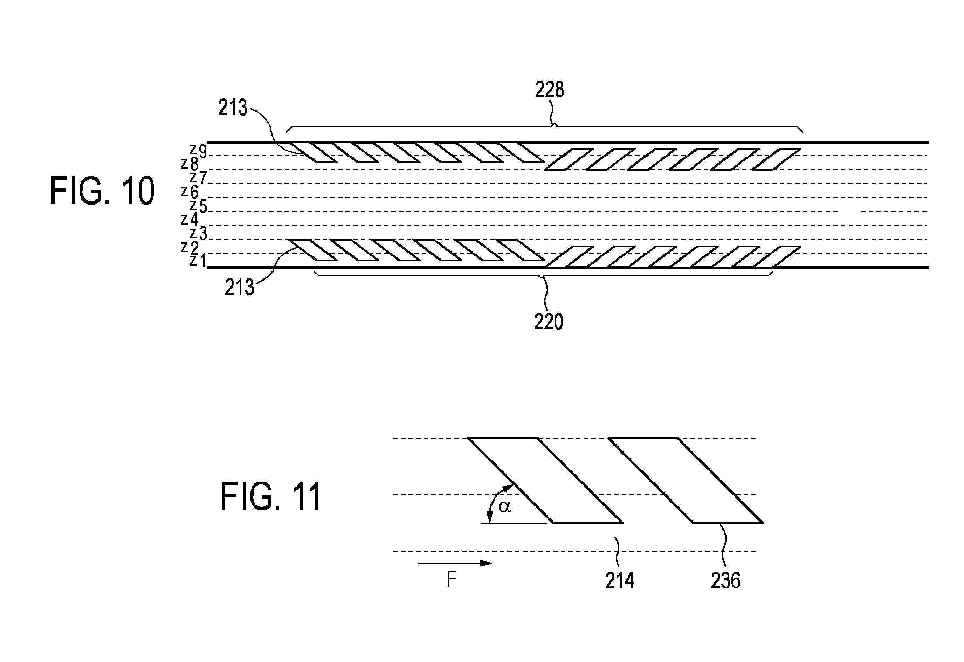

Figs. 8-10 illustrate arrangements of segments 224, 226, 228 where each ridged element 216, 218, 220 includes two rows of generally parallel ridged elements 212 arranged side-by-side. In addition, the arrangement of ridges of each segment 224, 226, 228 is slightly different. Viewed sequentially, each of the segments 224, 226, 228 includes the two generally parallel rows 213 of ridged elements 216, 218, 220 where the parallel rows 213 of the arrangement shown in Fig. 9 are spaced farther apart than in the arrangement shown in Fig. 8, and the arrangement shown in Fig. 10 includes wider spacing than in Fig. 9. Thus, if segments 224, 226, 228 are placed sequentially in a row in a single channel 202, each subsequent segment includes wider spacing between the parallel rows 213 than the preceding segment.

As explained previously, the purpose of the grooved/ridged elements 210, 216, 218, 220 indicated in Figs.

7-10 is to provide homogenization of the dissimilar fluids within the targeted zones, while the fluids in the other zones are not affected. Testing has shown this device to be effective in creating the desired results.

Fig. 11 illustrates a plan view of individual ridges 236. In one embodiment, the height of the ridged elements 210, 216, 218, 220 and each individual ridge 236 above a floor 214 of the channel 202 is approximately 20-40% of the total height of the channel 202. Each ridge 236 may include a profile that is squared to the channel floor 214 or each ridge 236 may have a rounded profile, depending on the desired fluid mixing effect desired. The individual ridges 236 and the ridged elements 210, 216, 218, 220 may be sized to provide an appropriate number of zones, and to accommodate the length and height limitations within the channel 202. In one embodiment, the width of each ridge 236 perpendicular to fluid flow F is approximately 74 $\mu$m and the length of each ridge 236 parallel to fluid flow F is approximately 50 $\mu$m, with a spacing of approximately 50 $\mu$m as well. However, the actual and relative sizes of the ridges 236 and ridged elements 210, 216, 218, 220 may be varied to suit the particular application. The ridges 236 of the ridged elements 210, 216, 218, 220 may be angled $\alpha$ with respect to the fluid flow direction F. The angle $\alpha$ may be within a range from 30˚ to 60˚.

As illustrated in Fig. 7, the first arrangement of ridges 238 in each segment 208, 224, 226, 228 may be angled clockwise from the fluid flow direction F, and the second arrangement 240 may be angled counterclockwise from the fluid flow direction F. This arrangement provides a localized mixing effect in one direction for the first half of the segment and a local mixing effect in a second direction for the second half of the segment. This arrangement may be reversed as well.

The gradient may be created with a wide variety of chemical fluid solutions, e.g., a concentrated antibiotic solution and a pure buffer solution.

The method of creating a concentration gradient with a microfluidic device 200 comprises constructing a microfluidic channel 202 that includes two or more fluid inlets 204, 206 and two or more channel segments 208, 224, 226, 228. At least one segment 208, 224, 226, 228 includes a ridged element 210, 216, 218, 220 that includes a plurality of ridges 213 in an interior channel wall 214. Fluid solutions may be introduced into the inlets 204, 206 and through the channel 202, to create a localized secondary flow effect and a local mixing effect in the fluid solutions via the ridged elements 210, 216, 218, 220 to form a concentration gradient.

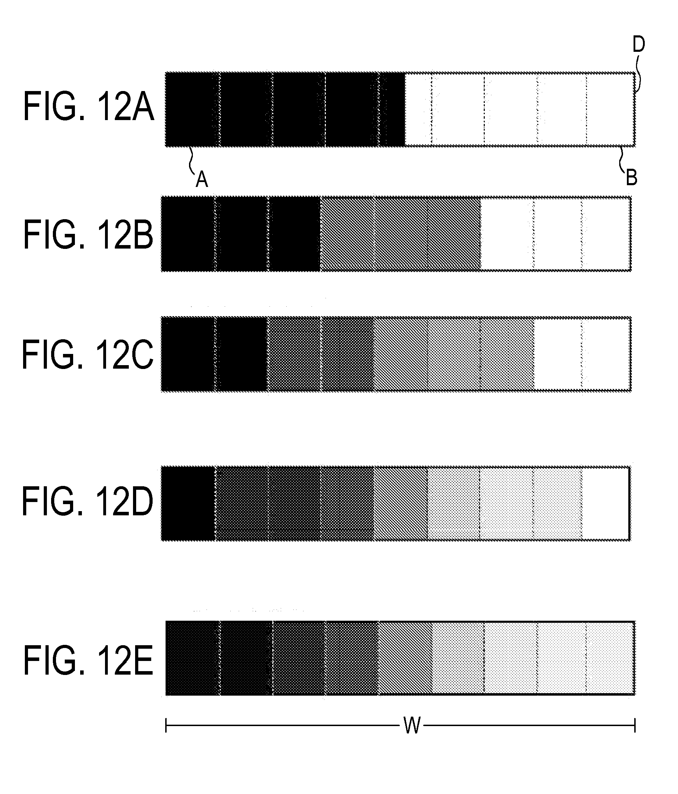

Fig. 12 provides exemplary concentration profiles of two fluids A, B at specific points along the channel 202, as a result of the localized mixing effects, according to the method presented. Fig. 12 illustrates the distribution and relative concentrations of two fluids across the width W of a channel 202. Fig. 12A illustrates the relative fluid arrangements immediately after the fluids enter the channel 202 at D, where there is no mixing between the two liquids at all. Figs. 12B-12E illustrate the relative fluid arrangements and concentrations after a number cycles, i.e., 10-15 cycles across the grooved/ridged elements 210, 216, 218, 220.

As the fluids A, B first enter the channel 202 at point D (see Fig. 7), very little mixing, if any, occurs. This fluid concentration profile, measured across the width W of the channel 202 is presented in Fig. 12A. Fluids A, B are side-by-side in a laminar flow, but have not been mixed. Diffusion is not a significant factor. Fig. 12B illustrates a concentration profile that would be observed immediately after the fluids A, B passed through segment 208. Fig. 7 illustrates a ridged element 210 in the middle of the channel 202 to provide a local mixing effect in the center of the channel 202. Thus, after passing through the first segment 208, only the center part of the fluid flow will be mixed to provide a partial concentration gradient as shown in Fig. 12B.

Next, the fluid passes through a second segment 224 having a ridged element 216 similar to that illustrated in Fig. 8. The ridged element 216 does not affect the fluid at the center of the channel 202 as the first segment 208, but affects the two new "boundaries" between the center fluid and the relatively unmixed fluids near the sides of the channel from Fig. 12B. Restated, the second step is the local mixing of the fluids A, B at the edges of the region homogenized in the first step. This is achieved with a grooved/ridged element 216 depicted in Fig. 8. Thus after the fluids A, B pass through the second segment 224, the overall appearance is a more gradual transition as illustrated in Fig. 12C.

Next, the fluid passes through a third segment 226 having a ridged element 218 similar to that illustrated in Fig. 9. The ridged element 218 does not affect the fluid in the same place as the second segment 226, but affects the two new "boundaries" between the previously mixed fluids and the relatively unmixed fluids near the sides of the channel from Fig. 12C. Thus after the fluids A, B pass through the third segment 226, the overall appearance is a more gradual transition as illustrated in Fig. 12D.

Finally, the fluid passes through a fourth segment 228 having a ridged element 220 similar to that illustrated in Fig. 10. The ridged element 220 does not affect the fluid in the same place as the third segment 228, but affects the two new "boundaries" between the previously mixed fluids and the relatively unmixed fluids near the sides of the channel from Fig. 12D. The third and fourth steps, illustrated in Figs. 12C-12D, results in mixing of the fluids in zones moved successively outward from previously homogenized zones, towards the side walls of the channel 202.

Thus after the fluids A, B pass through the fourth segment 228, the overall appearance is a more gradual transition as illustrated in Fig. 12E.

It is clear that, as illustrated in Figs. 12B-12E, the two fluids are mixed locally in the part of the channel 202 in which the grooved/ridged elements 210, 216, 218, 220 are present, but not away from the zones including the outside the grooved/ridged elements 210, 216, 218, 220. A local "homogenization" occurs, but not a general system-wide homogenization.

The eventual result of the local mixing in the four steps just described is shown in Fig. 12. As illustrated, the fluids are "homogenized" progressively, from the center of the channel 202 towards the side walls of the channel 202, leading to a linear concentration profile built up in a series of finite steps corresponding to the width of the zones defined in Fig. 7. To achieve this, it is important that the zones in which mixing/homogenization takes place in consecutive steps are overlapping partially.

Any number of segments, and inlets, may be assembled to create custom chemical gradients to meet the needs to a particular application. However, the goal is to create a predictable concentration gradient in a simple, repeatable manner. The sharpness of the chemical concentration gradient may be further adjusted based upon the concentrations of each of the chemicals introduced into the system. The dimensions used in the provided examples are specific, but the principle will also work with other dimensions. For the grooved/ridged elements 210, 216, 218, 220 to be effective, there are some dimension guidelines. The depth of the grooves/height of the ridges 236 should be between 20% and 40% of the channel height. The width of the grooves/ridges parallel to W should be between 50 % and 80% of the width W of the fluid stream to be homogenized. The streamwise dimension, parallel to fluid flow F, of the grooves/ridges should be smaller than their widthwise dimension. Depending upon the specific results desired, the total length of the channel 202 defined by the dimensions and number of repeats of the grooves/ridges, is 4x6,000=24,000 $\mu$m, i.e., about 50 times the channel width. Other geometrical designs of the grooved/ridged elements would lead to different concentration profiles at each step and/or overall, e.g., a non-linear concentration profile.

Valves may be used to swap between the two inlets enables to reverse the concentration profile, and may be done in an oscillatory way if needed. The induced concentration gradient is independent of the speed of fluid flow through the channel. This results because the secondary flow caused by the grooved/ridged elements 210, 216, 218, 220 scales with the streamwise flow speed. For very slow flows, diffusion may play a part in the general homogenization of the fluid over the channel width. This effect will be negligible, as long as the so-called Péclet-number Pe is large. Pe defined as $Pe = UL/D$, in which U is the characteristic flow velocity, L is the characteristic length scale, in this case the width of the channel, and D is the typical diffusion coefficient. Pe indicates the relative importance of convection to diffusion, and its value can be interpreted as the number of channel widths the fluid needs to travel in the channel direction to get complete mixing by diffusion. The diffusion coefficient of the buffer solution/growth factor solution can be estimated as $D \approx 1.10^{-11}$ $m^2$ $s^{-1}$. For L may be computed as the channel width W=500 $\mu$m, and for U the mean axial velocity. The condition Pe>100 (i.e. only after a travelling distance of 100 channel widths diffusion will play a significant role, recall that the total length for the specific case given above is 50 times the channel width) gives the condition U>10 $\mu$m/s. For flows with a lower mean speed, diffusion may come into play. This is an order-of-magnitude estimate that may take a different value for different channel dimensions.

Thus the microfluidic device 200 may be applied in situations in which a chemical concentration gradient of a species needs to be established, e.g., direct and fast determination of MIC, isoelectric point, pH-dependent adhesion or other concentration-dependent properties, in a single microfluidic chamber. The creation of a concentration gradient within the device does not rely strictly upon diffusion and may closely mimic a coronary artery for the study of chemotactics as well. A growth factor gradient may be used to measure chemotactic properties of cells. Another potential application is free flow iso-electrical focusing where a salt or conductivity gradient is required. Another potential application is DEP-focusing (Dielectrophoretic) where a conductivity gradient is required.

Thus, this invention presents a micro-fluidic device in which a concentration gradient of a (chemical) species is induced by geometrical patterns placed on the channel wall(s); a microfluidic system in which the concentration gradient can be reversed (continuously) in time by the integration of additional valves in the system;

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measured cannot be used to advantage.

Any reference signs in the claims should not be construed as limiting the scope.

**Claims**

1. A method for performing cellularsusceptibility testing via a concentration gradient of a substance in a micro fluidic device (200) including a microfluidic channel (202), comprising the steps of:

trapping one or more single cells to be tested in one or more defined positions on an inner surface of the channel (202);

applying a stable and predictable concentration gradient of a substance through the channel (202) with the cells to be tested;

incubating the cells to be tested within the concentration gradient, wherein, based upon the defined positions of the cells, an individual substance concentration at the location of each cell is known; and

determining a concentration-dependent property of the cells.

2. The method for performing cellularsusceptibility testing of claim 1, wherein at least one of the fluid solutions includes an antibiotic and the cells are bacteria.

3. The method for performing cellularsusceptibility testing of claim 1, wherein the cells are mammalian cells and the fluid is toxic to said cells.

4. The method for performing cellularsusceptibility testing of claim 2, further comprising the step of dynamically adjusting the concentration gradient of the antibiotic by varying the concentrations of at least one of two or more fluid solutions.

5. The method for performing cellularsusceptibility testing of claim 1, further comprising the step of dynamically adjusting the concentration gradient by varying the concentrations of at least one of two or more fluid solutions.

6. The method for performing cellularsusceptibility testing of claim 2, further comprising:

   incubating the bacteria within the concentration gradient with a viability stain; and
   determining the minimum inhibitory concentration and/or minimum bactericidal concentration from the defined positions of the bacteria within the stable and predictable antibiotic concentration gradient.

7. The method for performing cellularsusceptibility testing of claim 1, wherein the fluid solutions are selected to provide a pH and/or ionic strength gradient.

8. The method for performing cellularsusceptibility testing of claim 1, wherein the fluids flow into and through the channel (202) at a steady and/or variable rate.

9. The method for performing cellularsusceptibility testing of claims 6 or 7, wherein the bacteria are observed to determine an adhesion and/or isoelectric property with respect to the channel (202).

10. The method for performing cellularsusceptibility testing of claim 1, wherein at least one of two or more inlets (204, 206) includes at least one sub-inlet (205, 207) for introducing a fluid solution.

11. The method for performing cellularsusceptibility testing of claim 1, wherein the one or more single cells are trapped in one or more defined positions on an inner surface of the channel (202) with an adhesive.

12. A microfluidic device (250) for performing cellularsusceptibility testing via a concentration gradient comprising means for trapping (213) one or more single cells in one or more defined positions on an inner surface of the channel (202);

means for applying a stable and predictable fluid concentration gradient (210, 216, 218, 220) through the channel (202) with the cells;

means for incubating (260) the cells within the concentration gradient, wherein, based upon the defined positions of the cells, an individual fluid concentration for each cell is known; and

means for determining a concentration-dependent property (215) of the cells.

# FIG. 1A

# FIG. 1B

# FIG. 2A

302  304
306

300

312  310  308

# FIG. 2B

316

318

300  314  322  320

# FIG. 3

205

204  201  202

203

211

206  210

213

207

260

# FIG. 4

211

X

W

215

213

○ -DEAD

● -ALIVE

bacteria

# FIG. 5A

F

213

202

# FIG. 5B

F

X

215

213

202

# FIG. 6A

# FIG. 6B

# FIG. 7

EP 2 157 189 A1

EP 2 157 189 A1

**FIG. 8**

224

213

$z9$
$z8$
$z7$
$z6$
$z5$
$z4$
$z3$
$z2$
$z1$

222  213

216

**FIG. 9**

226

213

$z9$
$z8$
$z7$
$z6$
$z5$
$z4$
$z3$
$z2$
$z1$

213

218

FIG. 10

FIG. 11

FIG. 12A

FIG. 12B

FIG. 12C

FIG. 12D

FIG. 12E

Europäisches
Patentamt

European
Patent Office

Office européen
des brevets

# EUROPEAN SEARCH REPORT

Application Number

EP 08 10 5007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | CHENG SHING-YI ET AL: "A hydrogel-based microfluidic device for the studies of directed cell migration" LAB ON A CHIP, vol. 7, no. 6, June 2007 (2007-06), pages 763-769, XP002497558 ISSN: 1473-0197(print) 1473-0189(ele * the whole document * ----- | 1-12 | INV. C12Q1/18 B01L3/00 G01N33/50 |
| D,X | LIN FRANCIS ET AL: "T cell chemotaxis in a simple microfluidic device" LAB ON A CHIP, vol. 6, no. 11, 2006, pages 1462-1469, XP002497559 ISSN: 1473-0197(print) 1473-0189(ele * the whole document * ----- | 1-12 | |
| X | LI CHEUK-WING ET AL: "Dose-dependent cell-based assays in V-shaped microfluidic channels" LAB ON A CHIP, vol. 6, no. 7, 2006, pages 921-929, XP002497560 ISSN: 1473-0197(print) 1473-0189(ele * the whole document * ----- | 1-12 | |
| X | YANG M ET AL: "CELL DOCKING AND ON-CHIP MONITORING OF CELLULAR REACTIONS WITH A CONTROLLED CONCENTRATION GRADIENT ON A MICROFLUIDIC DEVICE" ANALYTICAL CHEMISTRY, AMERICAN CHEMICAL SOCIETY. COLUMBUS, US, vol. 74, no. 16, 15 August 2002 (2002-08-15), pages 3991-4001, XP001132776 ISSN: 0003-2700 * the whole document * ----- | 1-12 | |

TECHNICAL FIELDS SEARCHED (IPC)

C12Q
B01L
G01N

The present search report has been drawn up for all claims

| Place of search | Date of completion of the search | Examiner |
|---|---|---|
| Munich | 26 September 2008 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030040119 A1

**Non-patent literature cited in the description**

- **F. Lin ; E.C. Butcher.** *Lab Chip,* 2006, vol. 6, 1462-1469
- **Shing-Yi Cheng et al.** A Hydrogel-Based Microfluidic Device For The Studies Of Directed Cell Migration. *Lab On A Chip,* 2007, vol. 7, 763-769
- **Lam, E.W. ; Cooksey, G.A. ; Finlayson, B.A. ; Folch, A.** Microfluidic Circuits with Tunable Flow Resistances. *Applied Physics Letters,* 2006, vol. 89, 164105